# FASCICULE DE BREVET EUROPEEN

(11) **EP 1 664 044 B1**
(45) Date de publication et mention de la délivrance du brevet: **08.08.2007**
(21) Numéro de dépôt: 05778749.1
(22) Date de dépôt: 17.06.2005
(51) Int. Cl.: C07D 471/04

(54) **SEL DE SODIUM MONOHYDRATE DU S-TENATOPRAZOLE ET APPLICATION EN TANT QU'INHIBITEUR DE LA POMPE A PROTONS**
S-TENATOPRAZOLNATRIUM-MONOHYDRAT-SALZ UND DESSEN VERWENDUNG IN FORM EINES PROTONENPUMPENHEMMERS
S-TENATOPRAZOLE SODIUM MONOHYDRATE SALT AND THE USE THEREOF IN THE FORM OF A PROTON PUMP INHIBITOR

(30) Priorité: 17.06.2004 FR 0406617
(43) Date de publication de la demande: 07.06.2006
(73) Titulaire: Sidem Pharma, 2441 Luxembourg (LU)
(72) Inventeur: COHEN, Avraham, TEL AVIV (IL); SCHUTZE, François, F-78860 SAINT-NOM-LA-BRETECHE (FR); CHARBIT, Suzy, 94000 CRETEIL (FR); MARTINET, Frédéric, F-75002 PARIS (FR); FICHEUX, Hervé, F-94130 NOGENT-SUR-MARNE (FR); HOMERIN, Michel, F-91080 COURCOURONNES (FR)
(74) Mandataire: L'Helgoualch, Jean
(86) Numéro de dépôt international: PCT/FR2005/001528
(87) Numéro de publication internationale: WO 2006/005853

(56) Documents cités:
- EP-A1- 0 254 588
- WO-A1-20/04074285
- FR-A1- 2 848 555
- KAKINOKI B ET AL: "General pharmacological properties of the New Proton Pump Inhibitor (+-)-5-Methoxy -2-ÄÄ(4-Methoxy-3,5-dimethylpyrid-2-yl)met hylÜsulfinyl Ü-1H-imidazo Ä4,5-bÜpyridine" METHODS AND FINDINGS IN EXPERIMENTAL AND CLINICAL PHARMACOLOGY, PROUS, BARCELONA, ES, vol. 21, no. 3, 1999, pages 179-187, XP002979167 ISSN: 0379-0355

## Description

La présente invention concerne un sel de ténatoprazole, et plus particulièrement un sel monohydraté de l'énantiomère (-) du ténatoprazole, ou S-ténatoprazole, un procédé pour sa préparation, ainsi que son application en thérapeutique humaine ou vétérinaire, notamment comme inhibiteur de la pompe à protons pour le traitement du reflux gastro-oesophagien, des hémorragies digestives et des dyspepsies.

Divers dérivés de sulfoxydes, et notamment des pyridinyl-méthyl-sulfinyl benzimidazoles, ont été décrits dans la littérature pour leurs propriétés thérapeutiques permettant d'envisager leur utilisation comme médicaments présentant des propriétés inhibitrices de la pompe à protons, c'est-à-dire des médicaments qui inhibent la sécrétion d'acide gastrique et sont utiles pour le traitement des ulcères gastriques et duodénaux.

L'oméprazole, ou 5-méthoxy-2-[[(4-méthoxy-3,5-diméthyl-2-pyridinyl)méthyl]sulfinyl]-1H-benzimidazole décrit dans le brevet EP 005.129, est un des premiers dérivés connus de la série des inhibiteurs de la pompe à protons, qui possède des propriétés inhibitrices de la sécrétion acide gastrique, et est largement utilisé comme anti-ulcéreux en thérapeutique humaine. Parmi les autres dérivés connus des pyridinyl-méthyl-sulfinyl-benzimidazoles à structure similaire, on peut citer par exemple le rabéprazole, le pantoprazole, et le lansoprazole.

Le ténatoprazole, ou 5-méthoxy-2-[[(4-méthoxy-3,5-diméthyl-2-pyridyl)méthyl]sulfinyl]imidazo[4,5-b]pyridine, décrit dans le brevet EP 254.588, fait aussi partie des médicaments considérés comme des inhibiteurs de la pompe à protons, et il peut être utilisé dans le traitement du reflux gastro-oesophagien, des hémorragies digestives et des dyspepsies.

Ces sulfoxydes présentent une asymétrie au niveau de l'atome de soufre et peuvent donc se présenter sous forme de mélange racémique de deux énantiomères, ou sous forme de l'un ou l'autre des énantiomères. Ces énantiomères peuvent classiquement être utilisés sous forme de sels, tels que les sels de magnésium, de potassium ou de sodium, qui sont généralement plus faciles à mettre en oeuvre que les bases.

Le brevet EP 652.872 décrit le sel de magnésium de l'ésoméprazole, énantiomère (-) de l'oméprazole, ainsi qu'un procédé de préparation, séparation des diastéréo-isomères et solvolyse dans une solution alcaline. La préparation énantiosélective de l'énantiomère (-) de l'oméprazole ou de ses sels de sodium, par oxydation du sulfure correspondant par un hydroperoxyde en présence d'un complexe de titane et d'un ligand chiral est décrite dans le brevet US 5.948.789. Le procédé décrit dans ce brevet permet d'obtenir un mélange enrichi en l'un ou l'autre des énantiomères (-) et (+), selon le ligand utilisé.

Diverses formulations ont été proposées en vue d'améliorer les propriétés ou l'activité des inhibiteurs de la pompe à protons. Par exemple, la demande WO 01.28558 décrit une formulation liquide stable à base d'oméprazole, qui est obtenue en formant *in situ* les sels de sodium ou de potassium en solution dans le polyéthylène glycol, par action d'un hydroxyde sur l'oméprazole. Le médicament ainsi formulé est susceptible d'être utilisé dans les indications usuelles des inhibiteurs de la pompe à protons.

Des travaux récents ont montré que, contrairement à tous les autres inhibiteurs de la pompe à protons tels que, par exemple, l'oméprazole ou le lansoprazole, et de manière inattendue, le ténatoprazole possède une durée d'action nettement prolongée, résultant d'une demi-vie plasmatique environ 7 fois supérieure. Ainsi, les données médicales recueillies montrent que le ténatoprazole apporte un niveau de soulagement des symptômes et de cicatrisation des lésions gastriques supérieur à celui des autres médicaments appartenant à la même classe thérapeutique des inhibiteurs de la pompe à protons, ce qui permet alors son utilisation efficace dans le traitement des symptômes atypiques et oesophagiens du reflux gastro-oesophagien, des hémorragies digestives et des dyspepsies, comme indiqué ci-dessus. De plus, il a été montré que chacun des deux énantiomères (+) et (-), ou R et S respectivement, contribue de manière différente aux propriétés du ténatoprazole et que le S-ténatoprazole a des propriétés pharmacocinétiques significativement différentes de celles du racémique et de l'autre énantiomère. Le S-ténatoprazole est décrit dans la demande de brevet français 2.848.555 publiée le 18/06/2004.

Les travaux effectués par la demanderesse ont permis de montrer que le sel de sodium monohydraté du S-ténatoprazole présente des propriétés inattendues qui le distinguent du S-ténatoprazole lui même, et des autres inhibiteurs de la pompe à protons, et plus particulièrement une excellente solubilité facilitant les modalités de mise en forme pharmaceutique et améliorant significativement l'absorption et l'efficacité thérapeutique du médicament le contenant.

La présente invention a donc pour objet le sel de sodium monohydraté du S-ténatoprazole, et son utilisation en thérapeutique humaine ou vétérinaire.

Un autre objet de la présente invention est une solution concentrée de sel de S-ténatoprazole, et plus particulièrement une solution aqueuse dont la concentration en sel de sodium monohydraté de S-ténatoprazole est supérieure ou égale à 50 g/1, et de préférence supérieure ou égale à 100 g/l.

L'invention a également pour objet une composition pharmaceutique comprenant le sel de sodium monohydraté du S-ténatoprazole, substantiellement exempt de l'énantiomère (+) ou R-ténatoprazole, associé à un ou plusieurs excipients et supports pharmaceutiquement acceptables.

L'invention a aussi pour objet l'utilisation du sel de sodium monohydraté du S-ténatoprazole dans la fabrication d'un médicament pour le traitement de pathologies digestives où une inhibition de la sécrétion acide doit être intense et prolongée pour le traitement des symptômes et lésions du reflux gastro-oesophagien, des hémorragies digestives résistant aux autres inhibiteurs de la pompe à protons, ainsi que pour le traitement de ces maladies chez des patients polymédicamentés.

L'invention a aussi pour objet l'utilisation du sel de sodium monohydraté du S-ténatoprazole dans la fabrication d'un médicament procurant une amélioration significative de la cicatrisation ainsi qu'une augmentation de la vitesse de normalisation des modifications histologiques des lésions gastriques et oesophagiennes chez l'animal ou l'homme, et par conséquent une forte diminution des récidives.

L'invention a encore pour objet l'utilisation du sel de sodium monohydraté du S-ténatoprazole pour la fabrication d'un médicament ayant des propriétés pharmacocinétiques améliorées permettant une posologie d'une seule prise de médicament par jour dans les indications pertinentes, comme indiqué ci-après, en particulier pour l'éradication d'Helicobacter pylori dans le traitement des ulcères duodénaux, qui nécessitent deux prises, matin et soir, avec les autres inhibiteurs de pompe à protons.

L'invention a également pour objet un procédé de préparation énantiosélective du sel de sodium monohydraté du S-ténatoprazole, procurant le sel de l'énantiomère (-) avec une bonne pureté et un rendement satisfaisant.

Le sel de sodium monohydraté du S-ténatoprazole, peut être préparé par oxydation énantiosélective d'un sulfure de formule générale (I) ci-après

A - CH₂ - S - B (I)

dans laquelle A est un noyau pyridyle substitué et B un noyau imidazo-pyridyle,
au moyen d'un agent oxydant en présence d'un catalyseur à base de vanadium et d'un ligand chiral dans un solvant spécifique du sulfure et un solvant spécifique du ligand, conformément au procédé de préparation décrit dans la demande de brevet FR 2.863.611 , suivie d'une salification par l'hydroxyde de sodium.

Dans la formule générale (I) ci-dessus, le groupe pyridyle A est un groupe 4-méthoxy-3,5-diméthyl-2-pyridyle et B représente un groupe 5-méthoxy-imidazo[4,5-b]pyridyle.

L'oxydant utilisé est de préférence un peroxyde, et par exemple l'eau oxygénée. Suivant une forme avantageuse de réalisation, on utilise de préférence une eau oxygénée à concentration élevée, par exemple supérieure à 30%.

Le catalyseur peut être choisi parmi les catalyseurs tels qu'un complexe d'oxo-vanadium V et plus préférentiellement l'acétylacétonate de vanadium. De tels catalyseurs sont disponibles dans le commerce.

Un ligand tel qu'une base de Schiff dérivée d'un aldéhyde salicylique substitué et d'un amino-alcool chiral est utilisé de préférence en combinaison avec le catalyseur. Le choix du ligand permet d'orienter sélectivement la réaction vers l'énantiomère voulu. Ainsi, l'utilisation du 2,4-di-tert-butyl-6-[1-*R*-hydroxyméthyl-2-méthyl-propylimino)-méthyl]-phénol permet d'orienter sélectivement la réaction d'oxydation de la 5-méthoxy-2-[[4-méthoxy-3,5-diméthyl-2-pyridyl)méthyl]thio]-imidazo[4,5-b]pyridine, pour obtenir sélectivement le S-ténatoprazole.

La réaction est effectuée dans un solvant, et de préférence dans un mélange de solvants, en milieu neutre ou faiblement basique, en choisissant un solvant spécifique du sulfure et un solvant spécifique du ligand, choisis dans le groupe constitué par le méthanol, le tétrahydrofuranne, le dichlorométhane, l'acétonitrile, l'acétone et la N-méthyl-pyrrolidone ou le toluène, isolément ou en mélange. La base utilisée le cas échéant peut être une amine tertiaire telle que la pyridine, la di-isopropyléthylamine ou la triéthyl-amine. La création d'oxydation est réalisée aisément à froid ou à température ambiante.

Il est tout particulièrement avantageux d'utiliser le catalyseur à base de vanadium et le ligand en solution dans l'acétonitrile, tandis que le sulfure est en solution dans un solvant chloré tel que le dichlorométhane, et de réunir les deux solutions, puis de faire agir l'oxydant.

Plus particulièrement, l'oxydation du sulfure de formule (I) permet d'obtenir l'énantiomère (-), c'est-à-dire le S-ténatoprazole, dans d'excellentes conditions de pureté et de rendement en utilisant un catalyseur à base de vanadium associé à un ligand constitué par le 2,4-di-tert-butyl-6-[1-*R-*hydroxy-méthyl-2-methyl-propylimino)-méthyl]-phénol en solution dans l'acétonitrile, tandis que le sulfure est en solution dans le dichlorométhane. Dans les conditions opératoires, le ligand forme avec le catalyseur métallique un complexe asymétrique où le métal est oxydé par l'oxydant.

La réaction d'oxydation s'effectue aisément à froid ou à température ambiante, de préférence à une température comprise entre 0 et 10°C pour favoriser l'énantiosélectivité.

Le sulfure de formule (I) ci-dessus utilisé comme produit de départ est un produit connu qui peut être préparé par diverses méthodes décrites dans la littérature, et par exemple par les méthodes décrites dans les brevets EP 254.588 et EP 103.553.

On obtient ainsi le S-ténatoprazole, c'est-à-dire l'énantiomère lévogyre du ténatoprazole, qui peut être représenté par la formule développée suivante :

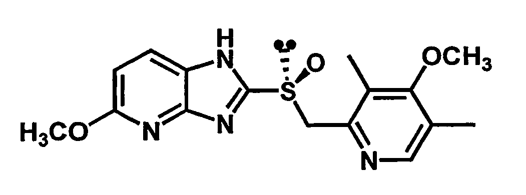

L'énantiomère (-) du ténatoprazole, ou S-ténatoprazole, correspond à la (-)-5-méthoxy-2-[[(4-méthoxy-3,5-diméthyl-2-pyridyl)méthyl]sulfinyl]imidazo[4,5-b]pyridine, ou (-)-ténatoprazole. Cette forme peut être déterminée par les mesures de rotation optique suivant les techniques usuelles. Ainsi, l'angle de rotation optique du (-)-ténatoprazole est lévogyre dans le diméthylformamide et dans l'acétonitrile, et son point de fusion est de 130°C (décomposition).

Suivant une variante, le S-ténatoprazole peut être obtenu sous forme optiquement pure à partir du mélange racémique, par des techniques bien connues, en utilisant une méthode de séparation appropriée, par exemple par chromatographie préparative sur colonne, comme la chromatographie chirale ou HPLC. Le principe de la méthode de chromatographie chirale repose sur la différence d'affinité existant entre chacun des énantiomères et le sélecteur chiral de la phase stationnaire.

Le mélange racémique utilisable comme matière de départ peut être obtenu par les procédés connus, par exemple suivant le procédé décrit au brevet EP 254.588. Ainsi, il peut être préparé en traitant par un agent oxydant, tel qu'un acide perbenzoïque, le sulfure correspondant provenant de la condensation d'un thiol et d'une pyridine. La réaction se fait de préférence en présence d'une base telle que l'hydroxyde de potassium dans un solvant approprié, par exemple l'éthanol, à chaud. Le mélange racémique ainsi obtenu peut être résolu par chromatographie HPLC comme indiqué ci-dessus.

Le S-ténatoprazole obtenu suivant l'une ou l'autre des méthodes ci-dessus est ensuite salifié pour obtenir le sel de formule (II) suivante :

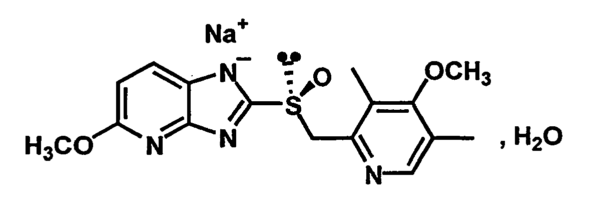

Dans la formule ci-dessus, l'atome de sodium peut être fixé sur le deuxième azote du noyau imidazopyridyle à proximité du groupe sulfoxyde, les deux isomères étant en équilibre.

La salification est réalisée par action d'hydroxyde de sodium, sur le S-ténatoprazole, à une température comprise entre 50 et 70°C, de préférence à environ 60°C, dans un solvant tel que l'eau, le chloroforme, le DMSO ou un solvant protique, par exemple le méthanol ou l'éthanol, puis en faisant précipiter le sel obtenu après élimination du solvant. La réaction est réalisée de préférence sous atmosphère inerte (azote ou argon) .

On fait précipiter le sel de manière classique en utilisant un solvant miscible à l'eau et dans lequel le sel est peu soluble, par exemple une cétone telle que l'acétone et la méthyl éthyl cétone. Le sel monohydraté peut être identifié par ses caractéristiques physico-chimiques, comme indiqué plus loin.

Le sel du ténatoprazole racémique peut être préparé par la même méthode afin d'effectuer des essais comparatifs, notamment de solubilité, avec le sel de sodium de l'énantiomère.

Les mesures d'analyse thermique et diffraction des rayons X ont permis de caractériser la structure du sel de sodium monohydraté du S-ténatoprazole, et ont montré l'existence du monohydrate du sel de sodium du S-ténatoprazole qui se différencie significativement des autres formes telles que la forme anhydre, la forme amorphe et les solvates.

Ainsi, d'autres phases cristallisées du sel de sodium peuvent être produites en modifiant les conditions de cristallisation (température, mode d'isolement) et les solvants (modulation de la polarité). Par exemple, l'utilisation de dioxanne aboutit à la formation d'un solvate du sel de sodium de l'isomère parfaitement cristallisé et caractérisé. La présence de dioxanne dans la maille cristalline semble toutefois inadaptée à l'usage pharmaceutique.

La forme amorphe, dont la préparation est décrite dans l'Exemple 6 ci-après, est non cristallisée, instable et difficilement utilisable dans les compositions pharmaceutiques.

Une autre phase cristallisée qui peut être obtenue est le sel de sodium anhydre, décrit dans l'Exemple 4 ci-après. Cependant, l'étude en DVS (Dynamic Vapor Sorption) a montré le caractère instable de ce polymorphe dans des conditions usuelles d'humidité relative, entraînant une déliquescence du produit. En raison de ce caractère d'instabilité, ce polymorphe n'est pas adapté à un usage pharmaceutique, en particulier dans des formulations usuelles.

Le profil thermogravimétrique du sel de sodium montre qu'une fraction variable d'eau (comprise entre 1 et 4%) se désorbe à basse température (de 30°C à 50°C) et constitue une fraction d'eau labile et réversible. La déshydratation d'une molécule d'eau est observée vers 130°C (environ 5% de perte en masse). Le sel de sodium monohydraté a également été caractérisé par DVS (Dynamic Vapor Sorption).

Comme indiqué plus haut, le sel de sodium monohydraté du ténatoprazole présente une excellente solubilité dans l'eau et les principaux solvants. Ainsi la solubilité dans l'eau est de l'ordre de 140 à 150 g/l à 25°C, et de 240 à 290 g/l à 45°C, qui est considérablement plus élevée que celle du sel de sodium du ténatoprazole racémique (env. 18 à 19 g/l), tandis que celles du ténatoprazole racémique et du S-ténatoprazole sont inférieures à 1 g/l.

Ce résultat est tout à fait inattendu par comparaison avec la solubilité des autres inhibiteurs de la pompe à protons bien connus.

Ainsi, le sel de sodium monohydraté du S-ténatoprazole permet de préparer des solutions très concentrées en principe actif médicamenteux, de concentration supérieure à 50 g/l, et de préférence supérieure à 100 g/l. A titre comparatif, le sel de sodium du racémique ne permet pas d'obtenir des concentrations supérieures à 19 g/l à température ambiante.

Le sel de sodium monohydraté du S-ténatoprazole présente de bonne caractéristiques de stabilité dans des conditions normales de température, de pression et d'hygrométrie. Suivant les conditions d'environnement et de conservation, le rapport stoechiométrique entre le sel de sodium et l'eau peut évoluer et être compris entre 1 et 2. Ainsi, des teneurs en eau correspondant aux formes sesquihydratées et dihydratées peuvent être détectées. Toutefois, ce phénomène est réversible. La présente demande concerne à la fois le sel de sodium monohydraté et les sels de sodium sesquihydratés et dihydratés du S-ténatoprazole.

Une étude chez le chien, a montré que l'utilisation du sel de sodium monohydraté du S-ténatoprazole permet d'obtenir une biodisponibilité très supérieure à celle obtenue avec le S-ténatoprazole, c'est à dire une concentration plus élevée (C max) ainsi qu'une exposition mesurée par l'aire sous courbe des concentrations en fonction du temps (AUC t) plus élevée pour une même dose. De plus, la libération plus rapide (Tmax 1,3 heure pour le sel de sodium monohydraté contre 2,5 heures pour le S-ténatoprazole) permet d'atteindre bien plus rapidement des concentrations thérapeutiques, et donc d'améliorer le délai d'action du médicament, favorisant ainsi les possibilités de traitement à la demande.

Ces résultats sont regroupés au tableau ci-dessous comparant le sel de sodium monohydraté (sel) au S-ténatoprazole de base (acide libre).

| dose | T max h | C max ng.ml⁻¹ | AUC t ng.h.ml⁻¹ |
|---|---|---|---|
| 100 mg/kg (sel) | 1,3 | 183 021 | 822 785 |
| 100 mg/kg (acide libre) | 2,5 | 104 751 | 434 017 |

L'amélioration permet de diminuer la dose administrée d'un facteur compris entre 1,5 et 2 pour une exposition similaire. Il en résulte que pour une même dose de principe actif, l'efficacité thérapeutique est doublée par l'utilisation du sel de sodium monohydraté selon la présente invention.

Une étude pharmacocinétique sur le chien (n=6) sur 4 semaines, comparant les effets du ténatoprazole racémique et du sel de sodium monohydraté du S-ténatoprazole a mis en évidence les propriétés originales de ce dernier.

Les résultats sont regroupés au tableau ci-dessous.

| dose | T max h | C max ng.ml⁻¹ | AUC t ng.h.ml⁻¹ |
|---|---|---|---|
| 5 mg/kg (sel) | 0,5 | 15 648 | 42 208 |
| 25 mg/kg (sel) | 0,5 | 77 548 | 148 633 |
| 50 mg/kg (sel) | 0,7 | 125 883 | 323 942 |
| 50 mg/kg Ténatoprazole racémique | 1,5 | 50 179 | 155 592 |

Dans ce tableau, les abréviations ont les significations usuelles c'est-à-dire que Cmax est la concentration plasmatique maximale, Tmax est le temps (durée) pour lequel la concentration plasmatique maximale est observée, AUCt est l'aire sous la courbe de la concentration plasmatique.

Ces résultats sont mesurés au 28^{ème} jour d'administration.

Dans cette étude, le sel de sodium monohydraté du ténatoprazole, aux doses de 5 mg/kg de poids, 25 mg/kg et 50 mg/kg, et le ténatoprazole racémique à la dose de 50 mg/kg, sont administrés sous forme de poudre en gélule.

Ces résultats montrent que le sel de sodium monohydraté a une action plus rapide (Tmax plus court) que le racémique quelle que soit la dose utilisée, et procure une valeur de l'AUC et du Cmax deux fois plus importante pour la même dose.

Ces résultats ont été confirmés par une étude clinique chez l'homme (n=6), au cours de laquelle on a successivement administré à des patients une dose unique constituée par : a) des gélules de sel de sodium monohydraté de S-ténatoprazole sous forme gastrorésistante suivant une technique classique, b) le même sel de sodium monohydraté conditionné en poudre (non gastroprotégée), et c) du ténatoprazole racémique non salifié, également en gélules contenant une poudre non gastroprotégée.

Les résultats obtenus sont regroupés dans le tableau ci-après.

| Formulation | Cmax ng.ml⁻¹ | AUCinf ng.h.ml⁻¹ | T1/2 h |
|---|---|---|---|
| a) | 5340 | 50844 | 7,81 |
| b) | 3199 | 31223 | 8,36 |
| c) | 2488 | 21058 | 7,29 |

AUC_{inf} est l'aire sous la courbe de la concentration plasmatique calculée de l'administration jusqu'à l'infini, avec extrapolation de la phase terminale, et T_{1/2} est la demi-vie plasmatique.

On constate donc que le sel de sodium monohydraté du ténatoprazole, même non gastroprotégé, apporte une amélioration significative des paramètres.

Ces résultats confirment ceux des études chez l'animal et démontrent que le sel de sodium monohydraté du S-ténatoprazole permet d'augmenter l'exposition (AUC) d'environ 50% par rapport au ténatoprazole racémique. Il en est de même pour la concentration maximale (Cmax).

Ainsi, le sel de sodium monohydraté du S-ténatoprazole non seulement possède des propriétés pharmacocinétiques différentes, mais permet de diminuer la dose d'environ un tiers pour une efficacité comparable.

Le sel de sodium monohydraté du S-ténatoprazole, dans le traitement des pathologies indiquées ci-après, peut être administré sous .les formes usuelles adaptées au mode d'administration choisi, par exemple par voie orale ou parentérale, de préférence par voie orale ou intraveineuse. En particulier, l'excellente solubilité du sel de sodium monohydraté du S-ténatoprazole permet de l'administrer par voie intraveineuse et d'assurer ainsi une biodisponibilité maximale du médicament.

Les formulations usuelles de la technique pharmaceutique peuvent être utilisées, et par exemple des comprimés ou des gélules contenant le sel de sodium monohydraté du S-ténatoprazole comme principe actif, ou encore des solutés buvables ou des émulsions ou solutions pour administration parentérale contenant le sel de sodium du ténatoprazole avec un support pharmaceutiquement acceptable usuel.

Suivant une forme avantageuse, on peut préparer des granulés gastrorésistants qui peuvent être insérés dans une gélule ou incorporés dans une formulation de comprimés. Les granulés gastrorésistants peuvent être par exemple préparés en appliquant une couche de polymère adapté, tel qu'un polymère cellulosique ou méthacrylique, et par exemple l'Eudragit® sur un noyau neutre portant une couche contenant le principe actif.

Suivant une autre forme particulièrement adaptée à la caractéristique de solubilité du sel de sodium monohydraté du S-ténatoprazole, le noyau est constitué d'un mélange de diluant, par exemple un diluant cellulosique, d'agent désintégrant, et de sel de sodium monohydraté de S-ténatoprazole, ce noyau étant revêtu d'une pellicule gastrorésistante, par exemple en acétophtalate ou en méthacrylate.

L'agent désintégrant peut être un polymère cellulosique, tel qu'un polymère de carboxyméthyl cellulose, par exemple la croscarmellose sodique. Le diluant utilisé est de préférence un excipient pour compression directe, qui évite le recours à une étape de granulation par voie humide. L'enrobage gastro-résistant peut être en Eudragit®.

Une telle formulation est conçue pour libérer le principe actif en moins de 25 minutes environ à pH 6,8, c'est-à-dire dans le duodénum après passage dans l'estomac à pH plus acide.

Suivant une autre caractéristique, le sel de sodium monohydraté présente une relative stabilité en milieu acide, qui le distingue des autres inhibiteurs de la pompe à proton. Cette propriété permet d'utiliser le sel de sodium monohydraté du S-ténatoprazole dans des formulations sans enveloppe gastrorésistante, selon le mode de traitement souhaité. De telles formulations présentent une pharmacocinétique optimisée et procurent un compromis idéal entre la libération du principe actif, son action immédiate et sa relativement faible dégradation dans l'estomac. Elles permettent ainsi de mettre à la disposition du praticien une alternative aux formulations gastrorésistantes décrites plus haut.

Le sel de sodium monohydraté du ténatoprazole peut être utilisé dans la fabrication de médicaments pour le traitement de pathologies digestives, en particulier celles où une inhibition de la sécrétion acide doit être intense et prolongée, pour le traitement des symptômes et lésions du reflux gastro-oesophagien, des hémorragies digestives résistant aux autres inhibiteurs de la pompe à protons.

La posologie est déterminée par le praticien en fonction de l'état du patient et de la gravité de l'affection. Elle est généralement comprise entre 10 et 120 mg, de préférence entre 10 et 80 mg, plus préférentiellement entre 15 et 40 mg, de principe actif par jour.

L'excellente solubilité du sel de sodium monohydraté du S-ténatoprazole permet d'assurer une meilleure absorption du principe actif, et donc une meilleure biodisponibilité.

En particulier, la biodisponibilité du principe actif dans une forme pour administration par voie orale, telle que comprimé ou gélule, est proche de celle obtenue par administration par voie intraveineuse, ce qui induit une grande efficacité du produit.

La préparation du sel de sodium monohydraté du S-ténatoprazole est décrite ci-après, ainsi que ses propriétés originales, afin d'illustrer la présente invention, sans en limiter la portée.

### Exemple 1

### Préparation du (S)-(-)-ténatoprazole

Dans un ballon de 5 L, on introduit 3 L de dichlorométhane puis 360 g de 5-méthoxy-2-[[4-méthoxy-3,5-diméthyl]-2-pyridyl)méthyl]thio]imidazo[4,5-b]pyridine. On laisse sous agitation à température ambiante pendant 30 min.

Dans un ballon de 2 L, on introduit successivement 700 mL d'acétonitrile, 5,22 g de 2,4-di-tert-butyl-6-[1-R-hydroxy-méthyl-2-methyl-propylimino)-méthyl]-phénol, puis 2,90 g de vanadyl acétylacétonate. Le mélange est agité à l'air, à température ambiante. Après 30 min d'agitation cette solution est additionnée à la précédente.

A ce mélange, sous agitation, on additionne 135 mL d'eau oxygénée à 30% pendant 20 h à température ambiante. Après séparation de la phase aqueuse, la phase organique est lavée deux fois à l'eau, puis séchée et concentrée sous pression réduite. On obtient 283 g de l'énantiomère recherché, avec un excès énantiomérique supérieur à 80% (rendement 75%). On effectue deux recristallisations successives dans un mélange méthanol/eau ou DMF/acétate d'éthyle et on obtient l'énantiomère avec un excès énantiomérique supérieur à 99%.
T_{F :} 127,5°C
[α]_{D} : -182 (c 0,1, DMF)
Spectre UV (méthanol-eau): λₘₐₓ: 272 nm (ε = 6180), 315 nm (ε = 24877).
Infra-rouge (KBr) : 3006, 1581, 1436, 1364, 1262 cm⁻¹.
RMN ¹³C (KOH, référence: 3-(triméthylsilyl)-1-propanesulfonate de sodium) δ (ppm) : 13,2 ; 15,0 ; 56,6 ; 60,8 ; 62,6 ; 107,2 ; 129,5 ; 130,4 ; 131,9 ; 135,1 ; 150,5 ; 151,4 ; 156,9 ; 160,7 ; 163,0 ; 166,6.
RMN ¹H (DMSO d₆, référence : TMS) δ (ppm) : 2,20 (s, 6H), 3,70 (s, 3H), 3,91 (s, 3H), 4,69-4,85 (m, 2H), 6,80 (d, J 8,5 Hz, 1H), 7,99 (d, *J* 8,5 Hz, 1 H), 8,16 (s, H), 13,92 (s, 1,H)

### Exemple 2

### Préparation du sel de sodium monohydraté du S-(-)-ténatoprazole

Dans un ballon de 50 ml équipé d'un agitateur, d'une sonde de température et d'un réfrigérant, on introduit 1,0 g de S-(-)-ténatoprazole obtenu comme indiqué dans l'Exemple 1, on ajoute 1,0 ml d'eau puis 0,6 ml d'hydroxyde de sodium en solution aqueuse (5 M) à température ambiante, en procédant sous agitation lente.

Le mélange réactionnel est porté à 60°C et maintenu sous agitation pendant 2,5 heures. On obtient un liquide huileux qui est refroidi à température ambiante, puis le solvant est éliminé sous pression réduite à 40°C dans un évaporateur rotatif. Après addition de 6 ml d'acétone et agitation, le produit de couleur jaune pâle précipite et est recueilli par filtration sur verre fritté puis rincé avec 2,0 ml d'acétone ou de diéthyl éther.

Après séchage à 40°C sous pression réduite pendant 20 h, on obtient 1,1 g de sel de sodium monohydraté du S-ténatoprazole avec un rendement supérieur à 90%.

La caractérisation du sel de sodium monohydraté a été faite par analyse thermique et par diffraction X.
Point de fusion T_{F} : 235°C (méthode des capillaires ; appareil Büchi B545)
Teneur en eau 5,8% (Karl Fischer)
Excès énantiomérique : supérieur à 99% (chromatographie chirale)
RMN ¹H (DMSO d₆, référence : TMS) δ (ppm) : 8.23 (1H, s) ; 7.70 (1H, d, J = 8.4 Hz) ; 6.37 (1H, d, J = 8.4 Hz) ; 4.73 (1H, d, . J= 12.9Hz) ; 4.37 (1H , d, J= 12.9Hz) ; 3.82 (3H, s) ; 3.70 (3H, s) ; 2.22 (3H, s) ; 2.21 (3H, s).
Analyse thermogravimétrique :

L'analyse thermogravimétrique est réalisée au moyen d'une thermobalance Netzsch SCA 409 PC/PG. Les mesures sont effectuées dans un creuset en aluminium, entre 20 et 150°C avec une vitesse d'échauffement de 2°/min sous atmosphère d'azote.

Le profil thermogravimétrique fait apparaître trois étapes successives :
- entre 10 et 40°C : évaporation, perte de 1,35% d'eau
- entre 90 et 130°C : déshydratation, perte de 4,65% (désorption d'une molécule d'eau)
- entre 160 et 230°C : dégradation, perte de 9,42%.
Diagramme de diffraction RX :

L'analyse a été faite au moyen d'un diffractomètre Siemens D5005 (anticathode en cuivre, tension 40 kV, intensité 30 mA, température ambiante, domaine de mesures de 3 à 30°, incrémentation entre chaque mesure 0,04°, temps de mesure par pas 4 s).

Le tableau des valeurs mesurées est indiqué ci-après :

| Sel de sodium monohydraté du S-Ténatoprazole | | | |
|---|---|---|---|
| Angle 2-Theta ° | valeur d (Angstrom) | Intensité (Coups) | Intensité (%) |
| 5,965 | 14,80418 | 508 | 2,9 |
| 6,585 | 13,41257 | 17768 | 100 |
| 10,389 | 8,50818 | 446 | 2,5 |
| 12,891 | 6,8615 | 1352 | 7,6 |
| 13,264 | 6,66969 | 670 | 3,8 |
| 15,341 | 5,77085 | 676 | 3,8 |
| 17,294 | 5,12337 | 507 | 2,9 |
| 19,247 | 4,60779 | 444 | 2,5 |
| 19,896 | 4,45871 | 1763 | 9,9 |
| 20,925 | 4,24174 | 740 | 4,2 |
| 21, 6 | 4,11076 | 627 | 3,5 |
| 21,824 | 4,06909 | 609 | 3,4 |
| 22,316 | 3,98048 | 484 | 2,7 |
| 22,885 | 3,88278 | 1106 | 6,2 |
| 23,457 | 3,78939 | 2731 | 15,4 |
| 25,479 | 3,49302 | 637 | 3,6 |
| 26,151 | 3,40479 | 864 | 4,9 |
| 26,636 | 3,34392 | 709 | 4 |
| 27,506 | 3,2401 | 380 | 2,1 |
| 28,32 | 3,14875 | 396 | 2,2 |
| 28,526 | 3,12648 | 467 | 2,6 |
| 29,708 | 3,00467 | 570 | 3,2 |

Le sel de sodium monohydraté a également été caractérisé par DVS (Dynamic Vapor Sorption).

Les enregistrements sont réalisés sur un appareil SMS (Surface Measurement System) ayant les caractéristiques suivantes :
- capacité maximum : 1,5 g
- sensibilité : 0,1 µg
- plage de température: 5 - 48°C
- plage d'humidité: 0 - 98% HR
- précision : 1% HR

Cette technique permet de déterminer l'aptitude d'un produit à s'hydrater, se déshydrater, se solvater et se désolvater en mesurant la prise ou la perte de masse en fonction de l'atmosphère contrôlée en teneur d'eau ou de solvant à une température moyenne.

Les résultats sont regroupés dans le tableau ci-après :

| Humidité résiduelle % | Prise en eau (p/p%) | |
|---|---|---|
| | Absorption | Désorption |
| 20 | 3.75 | 4.40 |
| 40 | 4.45 | 4.52 |
| 60 | 4.62 | 4.63 |
| 80 | 4.71 | 4.71 |

Ces résultats montrent que la stoechiométrie de la phase monohydratée est maintenue de 20 à 80% d'humidité relative sans apparition de phénomène de déliquescence, contrairement à la phase anhydre (voir Exemple 3). Ceci montre l'excellente stabilité du sel de sodium monohydraté en présence d'humidité.

Dans des conditions de forte humidité relative, supérieure à 80%, la stoechiométrie du sel de sodium du S-ténatoprazole peut évoluer, le nombre de molécules d'eau pouvant être compris entre 1 et 2. Cette forme, qui entre également dans le cadre de la présente invention, présente un diagramme de diffraction X analogue à celui présenté ci-dessus :

| Sel de sodium monohydraté du S-Tenatoprazole + Seconde hydratation partielle | | | |
|---|---|---|---|
| Angle 2-Theta ° | valeur d (Angstrom) | Intensité (Coups) | Intensité (%) |
| 5,921 | 14,91531 | 497 | 3 |
| 6,586 | 13,40893 | 16710 | 100 |
| 12,867 | 6,87461 | 1252 | 7,5 |
| 13,275 | 6,6642 | 675 | 4 |
| 17,269 | 5,13084 | 501 | 3 |
| 19,203 | 4,61808 | 590 | 3,5 |
| 19,941 | 4,44894 | 1967 | 11,8 |
| 20,999 | 4,22702 | 946 | 5,7 |
| 23,509 | 3,78109 | 1685 | 10,1 |
| 25,511 | 3,48876 | 457 | 2,7 |
| 26,262 | 3,39065 | 650 | 3,9 |
| 26,727 | 3,33264 | 729 | 4,4 |
| 27,544 | 3,23569 | 707 | 4,2 |
| 28,602 | 3,11837 | 471 | 2,8 |
| 29,765 | 2,99907 | 675 | 4 |

### Exemple 3

### Préparation du sel de sodium monohydraté du S-(-)-ténatoprazole

Suivant une variante du procédé de l'Exemple 2, on prépare le sel de sodium monohydraté en procédant comme indiqué ci-après.

Dans un ballon tricol de 250 ml muni d'un agitateur, d'un réfrigérant et d'une sonde de température, on charge 25 ml de chloroforme. On ajouter 10 g de S-Tenatoprazole obtenu comme indiqué dans l'Exemple 1, et on maintient sous agitation jusqu'à solubilisation dans le chloroforme. On refroidit sur bain eau / glace à 4-5°C, puis on ajoute 150 ml d'acétone et on maintient à 4-5°C.

On ajoute sous agitation 3.85 g de lessive de soude (30%) en maintenant la température à 4-5°C, puis on laisse le milieu réactionnel revenir à température ambiante (20-25°C) en maintenant le milieu sous agitation pendant 16 heures. On observe un début de précipitation après une heure de contact.

Le milieu réactionnel est refroidi à une température de 4-5°C sur bain de glace et maintenu sous agitation pendant 4 heures. Après filtration du milieu réactionnel sur verre fritté, on recueille une poudre qui est rincée avec 15 ml d'acétone préalablement glacé. Après séchage dans une étuve sous vide à 60°C une nuit, on obtient environ 10 g de produit sous forme de sel de sodium monohydraté du S-ténatoprazole avec un rendement supérieur à 90%.

Les caractéristiques du sel sont identiques à celles de l'Exemple 2.

### Exemple comparatif 4

### Préparation du sel de sodium anhydre du S-(-)-ténatoprazole

A partir du S-(-)-ténatoprazole de l'Exemple 1, et en procédant comme décrit dans l'Exemple 2, on fait réagir l'hydroxyde de sodium en solution aqueuse sur le S-ténatoprazole à 60°C pour obtenir un liquide huileux qui est repris dans l'acétone après élimination de l'eau sous pression réduite, puis rincé et séché. Le produit obtenu est mis en suspension dans un mélange méthanol / acétonitrile (25/75) à 50°C, puis on refroidit à 5°C pour former un précipité blanc qui est recueilli par filtration, en opérant à l'abri de l'humidité.

Rendement de cristallisation : 85%.
Le diagramme de diffraction RX, effectué sur un équipement de type Brüker D5000 (anticathode Cuivre, 40V 30mA), procure les résultats suivants :

| Angle (°) 2-Theta | valeur de d | Intensité |
|---|---|---|
| 6,6 | 13,3 | 100 |
| 9,5 | 9,3 | 1 |
| 14,3 | 6,2 | 2 |
| 15, 1 | 5,9 | 2 |
| 15,9 | 5,6 | 2 |
| 17,4 | 5,1 | 1 |
| 18,3 | 4,8 | 2 |
| 19,9 | 4,5 | 8 |
| 20,9 | 4,2 | 2 |
| 21,4 | 4,1 | 2 |
| 22,1 | 4,0 | 1 |
| 22,7 | 3,9 | 2 |
| 22,9 | 3,9 | 2 |
| 23,9 | 3,7 | 2 |
| 24,9 | 3,6 | 1 |
| 26,4 | 3,4 | 2 |
| 27,2 | 3,3 | 2 |
| 27,6 | 3,2 | 1 |
| 29,5 | 3,0 | 2 |
| 30,5 | 2,9 | 1 |
| 36,3 | 2,5 | 1 |

Caractéristiques de DVS (les enregistrements sont effectués dans les mêmes conditions que dans l'Exemple 2) :

| Humidité résiduelle % | Prise en eau (p/p%) | |
|---|---|---|
| | Absorption | Désorption |
| 0 | 0.00 | 3.41 |
| 20 | 0.12 | 12.09 |
| 40 | 0.25 | 16.45 |
| 60 | 0.65 | 19.14 |
| 80 | 24.86 | 24.86 |

On constate qu'au-delà de 60% d'humidité relative (humidité résiduelle) la phase anhydre devient déliquescente de manière irréversible, au contraire de la phase monohydratée.

### Exemple comparatif 5

### Préparation du solvate 1,4-dioxane / sel de sodium du S-(-)-ténatoprazole

A partir du S-(-)-ténatoprazole de l'Exemple 1, on fait réagir l'hydroxyde de sodium en solution aqueuse sur le S-ténatoprazole à 60°C suivant la méthode décrite dans l'Exemple 2, pour obtenir un liquide huileux qui est repris dans l'acétone après élimination de l'eau sous pression réduite, puis rincé et séché.

Le produit ainsi obtenu est mis en suspension dans un volume suffisant de 1,4-dioxane à 25°C (1 g pour environ 100 ml de dioxane). On laisse concentrer lentement la suspension à température ambiante pendant 48 heures puis on filtre pour obtenir le solvate 1,4-dioxane / sel de sodium (1/1) sous forme de poudre blanche.
Analyse thermogravimétrique :

L'analyse thermogravimétrique est réalisée dans les conditions décrites dans l'Exemple 2.

Dans une première étape on observe une évaporation.

Dans une deuxième étape, de 70 à 100°C, se produit la désolvatation du 1,4-dioxane. La perte de masse dans les 3ème et 4ème étapes justifie la stoechiométrie du solvate 1/1.
Diagramme de diffraction RX :

Le diagramme de diffraction RX est effectué sur un équipement de type Brüker D5000 (anticathode Cuivre, 40V 30mA). Les résultats sont indiqués ci-après.

| Angle (°) 2-Theta | Valeur de d | Intensité |
|---|---|---|
| 7,7 | 11,5 | 12 |
| 11, 5 | 7,7 | 39 |
| 12,6 | 7,0 | 100 |
| 13, 1 | 6,8 | 9 |
| 13,3 | 6,6 | 8 |
| 14,2 | 6,2 | 8 |
| 14,6 | 6,1 | 5 |
| 15,2 | 5,8 | 17 |
| 15,5 | 5,7 | 20 |
| 17,5 | 5,1 | 17 |
| 18,2 | 4,9 | 15 |
| 18,8 | 4,7 | 7 |
| 20,4 | 4,4 | 6 |
| 23,3 | 3,8 | 57 |
| 24,1 | 3,7 | 36 |
| 25,0 | 3,6 | 5 |
| 26,5 | 3,4 | 7 |
| 26,8 | 3,3 | 14 |
| 34,7 | 2,6 | 12 |
| 35,3 | 2,5 | 13 |
| 36,0 | 2,5 | 8 |

### Exemple comparatif 6

### Préparation du sel de sodium amorphe du S-(-)-ténatoprazole

Dans un ballon de 50 ml contenant 1,0 g de S-(-)-ténatoprazole obtenu comme indiqué dans l'Exemple 1, on ajoute 1,0 ml d'eau puis 0,6 ml d'hydroxyde de sodium en solution aqueuse (5 M) à température ambiante.

Le mélange réactionnel est porté à 60°C et maintenu sous agitation pendant 2,5 heures. On obtient un liquide huileux qui est refroidi à température ambiante, puis le solvant est éliminé sous pression réduite à 40°C. Après addition de 5 ml d'eau et agitation, le sel amorphe précipite et est recueilli par filtration. Le spectre de diffraction des rayons-X effectué ne présente pas de bande de diffraction.

## Revendications

1. Le sel de sodium monohydraté du S-ténatoprazole, représenté par la formule générale (II) ci-après .

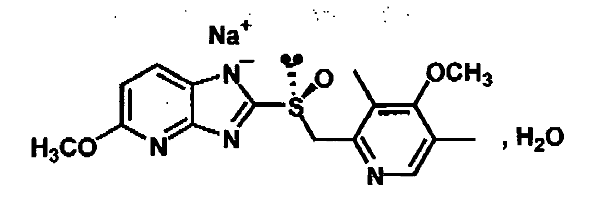

2. Solution concentrée de sel de sodium monohydraté de S-ténatoprazole, **caractérisée en ce que** la concentration en sel monohydraté est supérieure ou égale à 50 g/l

3. Solution concentrée selon la revendication 2, **caractérisée en ce que** la concentration en sel monohydraté est supérieure ou égale à 100 g/l.

4. Composition pharmaceutique **caractérisée en ce qu'**elle comprend le sel de sodium monohydraté du S-ténatoprazole selon la revendication 1, associé à un ou plusieurs excipients et supports pharmaceutiquement acceptables.

5. Composition selon la revendication 4, **caractérisée en ce qu'** elle est sous forme de dose unitaire contenant 10 à 80 mg de principe actif.

6. Composition selon la revendication 5, **caractérisée en ce que** la dose unitaire est comprise entre 15 et 40 mg.

7. L'utilisation du sel de sodium monohydraté du S-ténatoprazole substantiellement exempt de l'énantiomère (+) ou R-ténatoprazole, pour la fabrication d'un médicament pour le traitement des pathologies digestives.

8. L'utilisation du sel de sodium monohydraté du S-ténatoprazole pour la fabrication d'un médicament pour le traitement des pathologies digestives où une inhibition de la sécrétion acide doit être intense et:prolongée.

9. L'utilisation du sel de sodium du S-ténatoprazole pour la fabrication d'un médicament pour le traitement des pathologies digestives, du reflux gastro-oesophagien, et des hémorragies digestives, chez des patients polymédicamentés.

10. L'utilisation du sel de sodium monohydraté du S-ténatoprazole pour la fabrication d'un médicament présentant des propriétés pharmacocinétiques améliorées.

11. Procédé de préparation du sel de sodium monohydraté du S-ténatoprazole, **caractérisé en ce que** l'on fait agir l'hydroxyde de sodium sur le S-ténatoprazole, à une température comprise entre 50 et 70°C, puis on fait précipiter le sel obtenu après élimination du solvant.

12. Procédé selon la revendication 11, **caractérisé en ce que** la température réactionnelle est d'environ 60°C.

13. Procédé selon l'une quelconque des revendications 11 et 12, **caractérisé en ce que** la réaction est effectuée dans un solvant tel que l'eau, le chloroforme, le DMSO ou un solvant protique, par exemple le méthanol ou l'éthanol.

14. Procédé de préparation énantiosélective de sel de sodium monohydraté du S-ténatoprazole,
**caractérisé en ce qu'**il consiste à effectuer une oxydation énantiosélective d'un sulfure de formule générale (I) ci-après
A - CH₂ - S - B (I)
dans laquelle A est un groupe 4-méthoxy-3,5-diméthyl-2-pyridyle et B représente un groupe 5-méthoxy-imidazo[4,5-b]pyridyle,
au moyen d'un agent oxydant en présence d'un catalyseur à base de vanadium et d'un ligand chiral dans un solvant spécifique du sulfure et un solvant spécifique du ligand, suivie d'une salification par l'hydroxyde de sodium, pour obtenir le sel de sodium monohydraté du S-ténatoprazole.

15. Composition pour administration par voie orale du sel de sodium monohydraté du S-ténatoprazole, **caractérisée en ce qu'**elle est constituée d'un mélange de diluant, d'agent désintégrant, et de sel de sodium monohydraté de S-ténatoprazole, ce noyau étant revêtu d'une pellicule gastrorésistante.

16. Composition selon la revendication 15, **caractérisée en ce que** le diluant est un diluant cellulosique.

17. Composition selon la revendication 16, **caractérisée en ce que** le diluant est un excipient pour compression directe.

18. Composition selon la revendication 15, **caractérisée en ce que** l'agent désintégrant est un polymère cellulosique, tel qu'un polymère de carboxyméthyl cellulose.

19. Composition selon la revendication 18, **caractérisée en ce que** l'agent désintégrant est la croscarmellose sodique.

## Claims

1. The monohydrated sodium salt of S-tenatoprazole represented by the general formula (II) here-after:

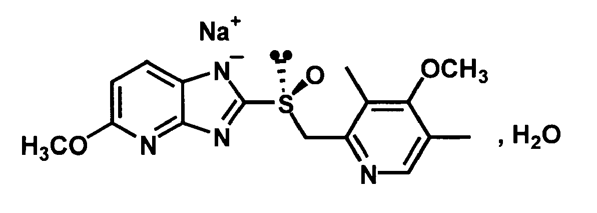

2. A concentrated solution of monohydrated sodium salt of S-tenatoprazole, wherein the concentration in monohydrated salt is higher than or equal to 50 g/l.

3. A concentrated solution according to claim 2, wherein the concentration in monohydrated salt is higher than or equal to 100 g/l.

4. A pharmaceutical composition comprising the monohydrated sodium salt of S-tenatoprazole according to claim 1, associated to one or more pharmaceutically acceptable excipients and substrates.

5. A composition according to claim 4, wherein it is under the form of unitary doses containing from 10 to 80 mg of active principle.

6. A composition according to claim 5, wherein the unitary dose is comprised between 15 and 40 mg.

7. The use of the monohydrated sodium salt of S-tenatoprazole substantially free from the (+) enantiomer or R-tenatoprazole, for the manufacture of a medicament for the treatment of digestive diseases.

8. The use of the monohydrated sodium salt of S-tenatoprazole for the manufacture of a medicinal product to treat digestive diseases where the inhibition of acid secretion must be effective and prolonged.

9. The use of the monohydrated sodium salt of S-tenatoprazole for the manufacture of a medicinal product to treat digestive diseases, gastro-oesophageal reflux disease and digestive bleeding in polymedicamented patients.

10. The use of the monohydrated sodium salt of S-tenatoprazole for the manufacture of a medicinal product exhibiting improved pharmacokinetic properties.

11. A method of preparation of the monohydrated sodium salt of S-tenatoprazole, wherein sodium hydroxide is caused to react on S-tenatoprazole at a temperature comprised between 50 and 70°C, and the salt obtained is precipitated after elimination of the solvent.

12. A method according to claim 11, wherein the reaction temperature is of about 60°C.

13. A method according to any of claims 11 and 12, wherein the reaction is conducted in a solvent such as water, chloroform, DMSO or a protic solvent, for example methanol or ethanol.

14. An enantioselective method of preparation of the monohydrated sodium salt of S-tenatoprazole, wherein an enantioselective oxidation is conducted on a sulphide of the following general formulation (I)
A - CH₂ - S - B (I)
where A is a 4-methoxy-3,5-dimethyl-2-pyridyl group and B represents a 5-methoxy-imidazo[4,5-b]pyridyl group,
using an oxidising agent in the presence of a vanadium based catalyst and a chiral ligand in a specific solvent of the sulphide and a specific solvent of the ligand, followed by salification by sodium hydroxide, in order to obtain the monohydrated sodium salt of S-tenatoprazole.

15. A composition for oral administration of the monohydrated sodium salt of S-tenatoprazole, wherein it consists of a mixture of a diluent, a disintegrating agent and the monohydrated sodium salt of S-tenatoprazole, this nucleus being covered with an enteric film.

16. A composition according to claim 15, wherein the diluent is a cellulosic diluent.

17. A composition according to claim 16, wherein the diluent is an excipient for direct compression.

18. A composition according to claim 15, wherein the disintegrating agent is a cellulosic polymer, such as a carboxymethyl cellulose polymer.

19. A composition according to claim 18, wherein the disintegrating agent is sodium croscarmellose.

## Patentansprüche

1. S-Tenatoprazolnatrium-monohydrat-Salz der nachstehenden allgemeinen Formel (II):

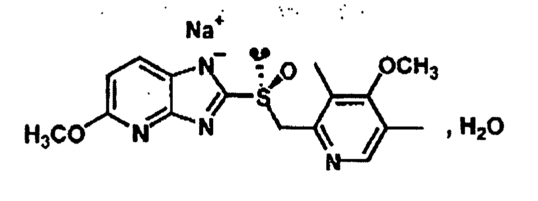

2. Konzentrierte Lösung des S-Tenatoprazolnatrium-monohydrat-Salzes, **dadurch gekennzeichnet, dass** die Konzentration des Monohydrat-Salzes 50 g/l oder mehr beträgt.

3. Konzentrierte Lösung nach Anspruch 2, **dadurch gekennzeichnet, dass** die Konzentration des Monohydrat-Salzes 100 g/l oder mehr beträgt.

4. Pharmazeutische Zusammensetzung, **dadurch gekennzeichnet, dass** sie das S-Tenatoprazolnatrium-monohydrat-Salz nach Anspruch 1 in Verbindung mit einem oder mehreren Arzneimittelträgern und pharmazeutisch annehmbaren Trägern umfasst.

5. Zusammensetzung nach Anspruch 4, **dadurch gekennzeichnet, dass** sie in Form einer Einheitsdosis vorliegt, die 10 bis 80 mg des Wirkstoffs umfasst.

6. Zusammensetzung nach Anspruch 5, **dadurch gekennzeichnet, dass** die Einheitsdosis zwischen 15 und 40 mg beträgt.

7. Verwendung des S-Tenatoprazolnatrium-monohydrat-Salzes, das im Wesentlichen frei vom (+)-Enantiomer oder R-Tenatoprazol ist, zur Herstellung eines Medikaments zur Behandlung von Erkrankungen des Verdauungstrakts.

8. Verwendung des S-Tenatoprazolnatrium-monohydrat-Salzes zur Herstellung eines Medikaments zur Behandlung von Erkrankungen des Verdauungstrakts, bei denen die Säureproduktion intensiv und für einen längeren Zeitraum zu hemmen ist.

9. Verwendung des S-Tenatoprazolnatrium-monohydrat-Salzes zur Herstellung eines Medikaments zur Behandlung von Erkrankungen des Verdauungstrakts, von gastroösophagealem Reflux und Blutungen im Verdauungstrakt bei Patienten, die mit mehreren Medikamenten behandelt werden.

10. Verwendung des S-Tenatoprazolnatrium-monohydrat-Salzes zur Herstellung eines Medikaments, das verbesserte pharmakokinetische Eigenschaften aufweist.

11. Verfahren zur Herstellung des S-Tenatoprazolnatrium-monohydrat-Salzes, **dadurch gekennzeichnet, dass** Natriumhydroxid auf S-Tenatoprazol bei einer Temperatur zwischen 50 und 70 °C einwirken gelassen wird, wonach das nach Entfernung des Lösungsmittels erhaltene Salz ausfallen gelassen wird.

12. Verfahren nach Anspruch 11, **dadurch gekennzeichnet, dass** die Reaktionstemperatur etwa 60 °C beträgt.

13. Verfahren nach Anspruch 11 oder Anspruch 12, **dadurch gekennzeichnet, dass** die Reaktion in einem Lösungsmittel, wie z.B. Wasser, Chloroform, DMSO oder einem protischen Lösungsmittel, wie z.B. Methanol oder Ethanol, durchgeführt wird.

14. Verfahren zur enantioselektiven Herstellung des S-Tenatoprazolnatrium-monohydrat-Salzes, **dadurch gekennzeichnet, dass** es darin besteht, eine enantioselektive Oxidation eines Sulfids der nachstehenden allgemeinen Formel (I)
A-CH₂-S-B (I)
worin A eine 4-Methoxy-3,5-dimethyl-2-pyridyl-Gruppe und B eine 5-Methoxyimidazo-[4,5-b]pyridyl-Gruppe ist, mit einem Oxidationsmittel in Gegenwart eines Katalysators auf Vanadiumbasis und eines chiralen Liganden in einem spezifischen Lösungsmittel für das Sulfid und einem spezifischen Lösungsmittel für den Liganden durchzuführen, wonach eine Salzbildung mit Natriumhydroxid erfolgt, um das S-Tenatoprazolnatrium-monohydrat-Salz zu erhalten.

15. Zusammensetzung zur oralen Verabreichung des S-Tenatoprazolnatrium-monohydrat-Salzes, **dadurch gekennzeichnet, dass** sie aus einem Gemisch aus einem Verdünnungsmittel, einem Zerfallsbeschleuniger und dem S-Tenatoprazolnatrium-monohydrat-Salz besteht, wobei dieser Kern mit einem magensaftresistenten Film beschichtet ist.

16. Zusammensetzung nach Anspruch 15, **dadurch gekennzeichnet, dass** das Verdünnungsmittel ein Cellulose-Verdünnungsmittel ist.

17. Zusammensetzung nach Anspruch 16, **dadurch gekennzeichnet, dass** das Verdünnungsmittel ein Arzneimittelträger zur direkten Kompaktierung ist.

18. Zusammensetzung nach Anspruch 15, **dadurch gekennzeichnet, dass** der Zerfallsbeschleuniger ein Cellulosepolymer, wie z.B. ein Carboxymethylcellulosepolymer, ist.

19. Zusammensetzung nach Anspruch 18, **dadurch gekennzeichnet, dass** der Zerfallsbeschleuniger Croscarmellosenatrium ist.
